# EUROPÄISCHE PATENTSCHRIFT

(11) **EP 0 548 103 B1**
(45) Veröffentlichungstag und Bekanntmachung des Hinweises auf die Patenterteilung: **05.12.2001**
(21) Anmeldenummer: 91914905.4
(22) Anmeldetag: 06.09.1991
(51) Int. Cl.: A61K 31/44

(54) **VERWENDUNG VON PYRIDYLMETHYLSULFINYL-1H-BENZIMIDAZOL DERIVATEN ZUR BEHANDLUNG DURCH HELICOBACTER VERURSACHTER ERKRANKUNGEN**
USE OF PYRIDYLMETHYLSULPHINYL-1H-BENZIMIDAZOLE DERIVATES IN THE TREATMENT OF ILLNESSES CAUSED BY HELICOBACTER BACTERIA
UTILISATION DE DERIVES DE PYRIDYLMETHYLSULFINYL-1H-BENZIMIDAZOL POUR LE TRAITEMENT DE MALADIES PROVOQUEES PAR HELICOBACTER

(30) Priorität: 14.09.1990 CH 299390; 25.07.1991 CH 222691
(43) Veröffentlichungstag der Anmeldung: 30.06.1993
(73) Patentinhaber: BYK GULDEN LOMBERG CHEMISCHE FABRIK GMBH, D-78403 Konstanz (DE)
(72) Erfinder: KLEMM, Kurt, D-7753 Allensbach (DE); KRÜGER, Uwe, D-7750 Konstanz (DE); STURM, Ernst, D-7750 Konstanz 18 (DE); SENN-BILFINGER, Jörg, D-7750 Konstanz (DE)
(86) Internationale Anmeldenummer: EP9101689
(87) Internationale Veröffentlichungsnummer: WO9204898

(56) Entgegenhaltungen:
- EP-A- 0 282 131
- EP-A- 0 382 489
- WO-A-89/11479
- WO-A-90/09175
- Antimicrobial Agents and Chemotherapy, Band 28. Nr. 6, Dezember 1985, C.A.A.McNulty et al.: " Susceptibility of clinical isolates of campylobacter pyloridis to 11 antimicrobial agents", Seiten 837-838, siehe Zusammenfassung; Seite 838, Tabelle 1
- European Journal of Clinical Microbiology & Infectious Diseases, Band 10, Nr. 2, February 1991, S. Suerbaum et al.: "Antibacterial activity of pantoprazole and omeprazole against helicobacter pylori", seiten 92-93, siehe das ganze Dokument

## Beschreibung

### Anwendungsgebiet der Erfindung

Die Erfindung betrifft neue orale Arzneiformen. Die neuen Arzneiformen werden zur Behandlung von Erkrankungen des Magens und/oder Darms eingesetzt, die durch Helicobacter-Bakterien hervorgerufen werden.

### Stand der Technik

In einer Vielzahl von Patentanmeldungen und Patenten werden Pyridylmethylsulfinyl-1H-benzimidazole beschrieben, die magensäuresekretionshemmende Eigenschaften besitzen. Im Zusammenhang mit der vorliegenden Erfindung seien hier insbesondere die folgenden Patentanmeldungen und Patente erwähnt: EP-A-134 400 (= USP 4,555,518), EP-A-127 763 (= USP 4,560,693), EP-B-166 287 (= USP 4,758,579), EP-A-201 575 (= USP 4,686,230), M089/05299 und W089/11479. - In der europäischen Patentanmeldung EP-A-382 489 wird die Eignung bestimmter Pyridylmethylsulfinyl-1H-benzimidazole, die im Benzimidazolteil gewünschtenfalls durch Methoxy oder Trifluormethyl substituiert sind, zur Behandlung infektiöser Erkrankungen, die durch Bakterien vom Stamme Campylobacter (= Helicobacter) hervorgerufen werden, beschrieben und beansprucht. In der internationalen Patentanmeldung M090/09175 wird die Verwendung von Omeprazol bei der Behandlung von infektiösen, insbesondere durch Campylobacter pylori hervorgerufenen Erkrankungen offenbart. - Aufgrund der geringen Stabilität und leichten Säurezersetzlichkeit der Pyridylmethylsulfinyl-1H-benzimidazole wird in verschiedenen Patentanmeldungen (z.B. EP-A-244 380 oder EP-A-247 983) auf die Notwendigkeit verwiesen, bei oraler Applikation diese Wirkstoffe in einer magensaftresistenten Form zu verabreichen. Auch in der obengenannten EP-A-382 489 wird als orale Darreichungsform für die Campylobacter-Bekämpfung beispielhaft eine "enteric coated" Formulierung verwendet.

### Beschreibung der Erfindung

Gegenstand der Erfindung ist die Verwendung von 5-Difluormethoxy-2-[(3,4-dimethoxy-2-pyridyl)methylsulfinyl]-1H-benzimidazo] und seinen pharmakologisch verträglichen Salzen zur Herstellung von oral zu verabreichenden Arzneimitteln für die Bekämpfung von Helicobacter-Bakterien.

Als Salze kommen bevorzugt pharmakologisch verträgliche basische Salze in Betracht, insbesondere pharmakologisch verträgliche Salze mit in der Galenik üblicherweise verwendeten anorganischen und organischen Basen. Als Beispiele für basische Salze seien Lithium-, Natrium-, Kalium-, Calcium-, Aluminium-, Magnesium-, Titan-, Ammonium- oder Guanidiniumsalze erwähnt.

Von den Helicobacter-Stämmen sei insbesondere der Stamm Helicobacter pylori erwähnt.

Als oral zu verabreichende Arzneimittel seien beispielsweise Tabletten, Dragees, harte und weiche Kapseln, z.B. aus Gelatine, dispergierbare Pulver, Granulate, wäßrige und ölige Suspensionen, Emulsionen, Lösungen oder Sirupe erwähnt, wobei die Tabletten, Dragees, Kapseln oder Granulate vorteilhafterweise so beschaffen sind, daß sie sich im Magensaft leicht auflösen und den Wirkstoff im Magen freigeben.

Zur kombinierten Behandlung von Magenerkrankungen, die sowohl auf einer erhöhten Magensäuresekretion, als auch auf einer Schädigung des Magens durch Helicobacter pylori beruhen, seien auch solche oral zu verabreichenden Arzneiformulierungen erwähnt, die in einer Einzeldosis Wirkstoff gleichzeitig sowohl in magensaftresistenter, als .auch in nicht magensaftresistenter.Form enthalten. Beispielsweise seien genannt Tabletten, die den Wirkstoff sowohl in einem magensaftresistenten Kern, als auch in einer nicht magensaftresistenten Hülle enthalten, oder Kapseln, die mit magensaftresistenten und nicht magensaftresistenten Pellets oder (Mini)tabletten gefüllt sind.

Im allgemeinen wird in der Humanmedizin der Wirkstoff in einer Tagesdosis von etwa 0,05 bis etwa 5, vorzugsweise 0,1 bis 2,5 mg/kg Körpergewicht, gegebenenfalls in Form mehrerer, vorzugsweise 2 bis 6 Einzelgaben zur Erzielung des gewünschten Ergebnisses verabreicht.

Überraschenderweise wurde gefunden, daß die Verbindung 5-Difluormethoxy-2-[(3,4-dimethoxy-2-pyridyl)methylsulfinyl]-1H-benzimidazol in saurem Milieu gegen Helicobacter-Bakterien wesentlich wirksamer ist als in neutralem Milieu, und daß sie demzufolge - entgegen der aus dem Stand der Technik zu entnehmenden Lehre - sinnvollerweise nicht in einer magensaftresistenten Form verabreicht werden sollte.

Gegenstand der Erfindung ist somit die Verwendung von 5-Difluormethoxy-2-[(3,4-dimethoxy-2-pyridyl)methylsulfinyl]-1H-benzimidazol und seinen pharmakologisch verträglichen Salzen zur Herstellung von oral zu verabreichenden, nicht magensaftresistent formulierten Arzneimitteln für die Bekämpfung von Helicobacter-Bakterien.

Die Verbindung 5-Difluormethoxy-2-[(3,4-dimethoxy-2-pyridyl)methylsulfinyl]-1H-benzimidzol und ihre pharmakologisch verträglichen Salze sind aus dem Patent EP-B-166 287 bekannt.

Die Herstellung der oral zu verabreichenden Arzneimittel unter Verwendung der Wirkstoffe der Formel I erfolgt in einer dem Fachmann an sich bekannten Weise.

## Patentansprüche

1. Verwendung von 5-Difluormethoxy-2-[(3,4-dimethoxy-2-pyridyl)methylsulfinyl]-1H-benzimidazol und seinen pharmakologisch verträglichen Salzen zur Herstellung von oral zu verabreichenden Arzneimitteln für die Bekämpfung von Helicobacter-Bakterien.

2. Verwendung von 5-Difluormethoxy-2-[(3,4-dimethoxy-2-pyridyl)methylsulfinyl]-1H-benzimidazol-Natrium zur Herstellung von oral zu verabreichenden nicht magensaftresistent formulierten Arzneimitteln für die Bekämpfung von Helicobacter-Bakterien der Spezies Helicobacter pylori.

3. Arzneiformulierung enthaltend 5-Difluormethoxy-2-[(3,4-dimethoxy-2-pyridyl)methylsulfinyl]-1H-benzimidazol oder ein pharmakologisch verträgliches Salz davon gleichzeitig in magensaftresistenter Form als auch in nicht magensaftresistenter Form.

4. Arzneiformulierung nach Anspruch 3 in Form von Tabletten enthaltend den Wirkstoff in einem magensaftresistenten Kern und einer nicht magensaftresistenten Hülle.

5. Arzneiformulierung nach Anspruch 3 in Form von Kapseln enthaltend den Wirkstoff in magensaftresistenten und nicht magensaftresistenten Pellets oder (Mini)tabletten.

## Claims

1. Use of 5-difluoromethoxy-2-[(3,4-dimethoxy-2-pyridyl)methylsulphinyl]-1 H-benzimidazole and its pharmacologically tolerated salts for the preparation of medicaments to be administered orally for combating Helicobacter bacteria.

2. Use of 5-difluoromethoxy-2-[(3,4-dimethoxy-2-pyridyl)methylsulphinyl]-1H-benzimidazole sodium for the preparation of medicaments which are not in a formulation which is resistant to gastric juice and are to be administered orally for combating Helicobacter bacteria of the species Helicobacter pylori.

3. Drug formulation containing 5-difluoromethoxy-2-[(3,4-dimethoxy-2-pyridyl)methylsulphinyl]-1 H-benzimidazole or a pharmacologically tolerated salt thereof simultaneously in a form which is resistant to gastric juice and in a form which is not resistant to gastric juice.

4. Drug formulation according to claim 3 in the form of tablets which contain the active compound both in a core which is resistant to gastric juice and in a shell which is not resistant to gastric juice.

5. Drug formulation according to claim 3 in the form of capsules which contain the active compound in pellets or (mini)tablets which are both resistant to gastric juice and not resistant to gastric juice.

## Revendications

1. Utilisation de 5-difluorométhoxy-2-[(3,4-diméthoxy-2-pyridyl)méthylsulfinyl]-1H-benzimidazol et de ses sels pharmacologiquement acceptables pour la fabrication de médicaments susceptibles d'être administrés par voie orale, destinés à combattre des bactéries Helicobacter.

2. Utilisation de 5-difluorométhoxy-2-[3,4-diméthoxy-2-pyridyl)méthylsulfinyl]-1H-benzimidazol-sodium pour la fabrication de médicaments susceptibles d'être administrés par voie orale sous une forme ne résistant pas au suc gastrique, destinés à combattre des bactéries Helicobacter de l'espèce Helicobacter pylori.

3. Médicament contenant du 5-difluorométhoxy-2-[(3,4-diméthoxy-2-pyridyl)méthylsulfinyl]-1H-benzimidazol ou un sel pharmacologiquemen acceptable de celui-ci à la fois sous une forme résistant au suc gastrique et sous une forme ne résistant pas au suc gastrique.

4. Médicament selon la revendication 3, sous forme de comprimés contenant le principe actif sous forme d'un noyau résistant au suc gastrique et d'une enveloppe ne résistant pas au suc gastrique.

5. Médicament selon la revendication 3 se présentant sous forme de gélules contenant le principe actif sous forme de granulés ou (mini)comprimés résistant au suc gastrique et de granulés ou (mini)comprimés ne résistant pas au suc gastrique.
